# EUROPÄISCHE PATENTANMELDUNG

(11) **EP 0 989 126 A1**
(43) Veröffentlichungstag der Anmeldung: **29.03.2000**
(21) Anmeldenummer: 99117241.2
(22) Anmeldetag: 02.09.1999
(51) Int. Cl.: C07D 311/72

(54) **Verfahren zur Herstellung von Chromanol-Derivaten**

(30) Priorität: 23.09.1998 DE 19843672
(71) Anmelder: BASF AKTIENGESELLSCHAFT, 67056 Ludwigshafen (DE)
(72) Erfinder: Baldenius, Kai-Uwe, Dr., 67227 Frankenthal (DE); Bockstiegel, Bernhard, Dr., 67354 Römerberg (DE); Jaedicke, Hagen, Dr., 67069 Ludwigshafen (DE); Ruff, Detlef, Dr., 67067 Ludwigshafen (DE); Siedenbiedel, Carsten, Dr., 68165 Mannheim (DE); Stürmer, Rainer, Dr., 67127 Rödersheim-Gronau (DE)

(57) **Zusammenfassung**

Die Erfindung betrifft ein Verfahren zur Herstellung von Chromanol-Derivaten der allgemeinen Formel I 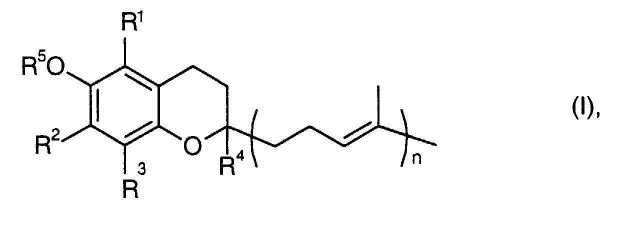 wobei
- n: 1 bis 10,
- R^{1,} R^{2,} R^{3,} R⁴: unabhängig voneinander Wasserstoff oder einen C₁-C₄-Alkylrest,
- R⁵: Wasserstoff, einen C₁-C₄-Alkyl-, C₆-C₁₀-Aryl-, C₇-C₁₈-Aralkyl-, C₇-C₁₈-Alkylaryl-, C₁-C₂₂-Acylrest oder eine Schutzgruppe der Hydroxygruppe
bedeuten, durch Reduktion der entsprechenden 4-Oxo-Chromanol-Derivate der allgemeinen Formel II 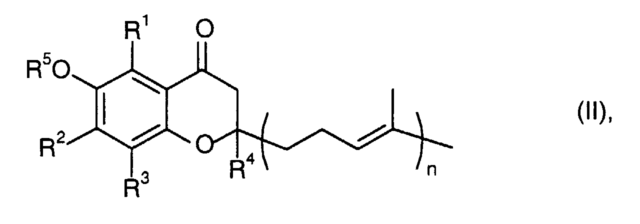 indem man die 4-Oxo-Chromanol-Derivate der Formel II mit metallischem Zink in Gegenwart einer Säure oder einer Säuremischung umsetzt.
Verbindungen der Formel I mit einem C₁-C₂₂-Acylrest als Rest R⁵ können auch durch vorstehend beschriebene Reduktion der Verbindungen der Formel II mit Wasserstoff als Rest R⁵ und gleichzeitiger oder anschließender Veresterung hergestellt werden.

## Beschreibung

Die Erfindung betrifft ein Verfahren zur Herstellung von Chromanol-Derivaten mit olefinischen Seitenketten durch Reduktion der entsprechenden 4-Oxo-Chromanolderivate.

Chromanole mit isoprenoiden Seitenketten, wie die Gruppe der Tocopherole und Tocotrienole zeigen Vitamin-E-Aktivität und sind daher als biologische Wirkstoffe von Bedeutung. Die Herstellung von Chromanol-Derivaten mit olefinischen, isoprenoiden Seitenketten wie Tocotrienol ist jedoch aufwendig, da die z.B. bei der Herstellung von Tocopherol üblichen sauren Synthesebedingungen (Ullmann's 5^{th} Ed 1996, Vol. A27, Chapter 4.11.2, S. 484-485) zur Isomerisierung oder Cyclisierung der olefinischen Seitenkette führen (P. Karrer, H. Reutschler, *Helv*. *Chim*. *Acta* **1944**, *27*, 1297; H.J. Kabbe, A. Widdig, *Angew*. *Chem*. *Int*. *Ed*. *Engl*. **1982**, *21*, 247-256, P. Schudel, H. Mayer, J. Metzger, R. Rüegg, O. Isler, *Helv*. *Chim*. *Acta* **1963**, *46*, 2517).
Es ist bekannt, Tocotrienole durch Reduktion der entsprechenden 4-Oxo-Tocotrienole herzustellen, die durch ein an sich bekanntes Verfahren zugänglich sind (H. J. Kabbe, A. Widdig, *Angew*. *Chem*. *Int*. *Ed*. *Engl*. **1982**, *21*, 247-256; H. J. Kabbe, H. Heitzer, Synthesis **1978**, 888).
Es ist bekannt, die Reduktion in einem mehrstufigen Verfahren, mit Natrium-boranat zu 4-Hydroxy-Tocotrienol, anschließender Wasserabspaltung durch Destillation und weiterer Partialhydrierung mit Na/Ethanol durchzuführen (H.J. Kabbe, H. Heitzer, *Synthesis* **1978**, 888). Durch den mehrstufigen Prozeß ist dieses Verfahren sehr aufwendig.
Weiterhin ist bekannt, die Reduktion in einem einstufigen Verfahren durch Verwendung komplexer Aluminiumhydride durchzuführen (B. C. Pearce et al., *J. Med. Chem*. **1994**, *37*, 526-541). Die Verwendung der komplexen Hydride im technischen Maßstab ist unwirtschaftlich und erfordert besonders hohe Sicherheitsvorkehrungen.

Es stellte sich daher die Aufgabe, den geschilderten Mängeln abzuhelfen und ein einfaches einstufiges Reduktionsverfahren zu entwickeln, die 4-Oxo-Chromanole mit olefinischer, isoprenoider Seitenkette zu den entsprechenden Chromanol-Derivaten zu reduzieren, ohne die Doppelbindungsgeometrie oder -lage in der ungesättigten Seitenkette durch Isomerisierung oder Cyclisierung zu verändern.

Demgemäß wurde gefunden, daß man die Chromanolderivate der allgemeinen Formel I 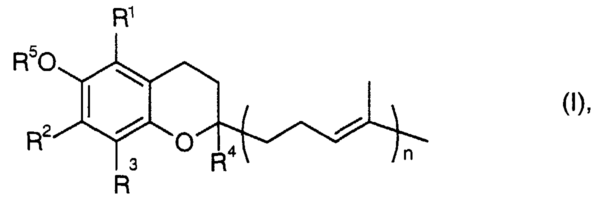 wobei
- n: 1 bis 10,
- R^{1,} R^{2,} R^{3,} R⁴: unabhängig voneinander Wasserstoff oder einen C₁-C₄-Alkylrest,
- R⁵: Wasserstoff, einen C₁-C₄-Alkyl-, C₆-C₁₀-Aryl-, C₇-C₁₈-Aralkyl-, C₇-C₁₈-Alkylaryl-, C₁-C₂₂-Acylrest oder eine Schutzgruppe der Hydroxygruppe
bedeuten, durch Reduktion der entsprechenden 4-Oxo-Chromanolderivate der allgemeinen Formel II 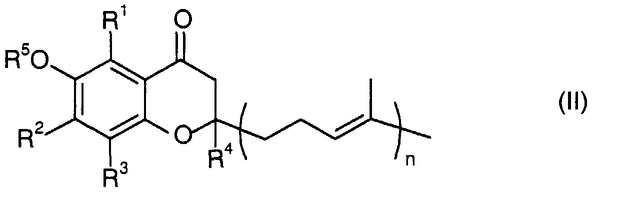 herstellt, indem man die 4-Oxo-Chromanolderivate mit metallischem Zink in Gegenwart einer Säure oder einer Säuremischung umsetzt.

Die an sich bekannte Clemmensen-Reduktion (R.R. Read et al, *Org*. *Synth. Collect*. Vol. 3, **1955**, 444; P. S. Bramwell, *J. Chem. Soc*. **1965**, 3882) ist für die Lösung der Aufgabe bislang nicht in Betracht gezogen worden, da die für die Clemmensen-Reduktion üblichen sauren Reaktionsbedingungen mit der säurelabilen, olefinischen Seitenkette nicht kompatibel sind (H. J. Kabbe, H. Heitzer, *Synthesis* **1978**, 888). Es wurde gefunden, daß es mit dem erfindungsmäßigen Verfahren gelingt, 4-Oxo-Chromanole mit ungesättigter Seitenkette zu reduzieren, ohne daß die olefinische Seitenkette isomerisiert oder cyclisiert.

Edukte des Verfahrens sind die 4-Oxo-Chromanolderivate der allgemeinen Formel II. Die olefinische, isoprenoide Seitenkette kann aus einer bis zehn Isopren-Einheiten bestehen (n = 1 bis 10).

Bevorzugte Edukte sind 4-Oxo-Chromanolderivate mit n = 2 bis 4 Isopreneinheiten, besonders bevorzugt die Gruppe der 4-Oxo-Tocotrienole mit n = 3 Isopreneinheiten.
Die Reste R¹, R², R³ und R⁴ können dabei unabhängig voneinander Wasserstoff oder C₁-C₄-Alkyl wie Methyl, Ethyl, n-Propyl, i-Propyl, n-Butyl, i-Butyl oder t-Butyl bedeuten, wobei Wasserstoff und Methyl bevorzugt sind.
R⁵ stellt Wasserstoff, einen C₁-C₄-Alkylrest, wie vorstehend beschrieben, einen C₆-C₁₀-Arylrest, wie Phenyl, einen C₇-C₁₈-Aralkylrest, wie Benzyl, Phenylethyl, einen C₇-C₁₈-Alkylarylrest, wie Tolyl, Mesityl oder einen C₁-C₂₂-Acylrest, wie einen C₁-C₂₂-Alkylacylrest, insbesondere Formiat, Acetat, Propionat, Butyrat, Pentanoat, Hexanoat, Heptanoat, 2-Ethylhexanoat, Octanoat, Nonanoat, Decanoat, Undecanoat, Laurinat, Palmitat, oder Stearat oder wie einen olefinischen C₂-C₂₂-Acylrest, insbesondere Acrylat, Methacrylat, Crotonat, Sorbinoat, 9-Octadecenoat, Linolat, Linolenat, Eicosapentaenoat oder Docosahexaenoat dar.

Ferner kann R⁵ eine übliche Schutzgruppe der Hydroxygruppe darstellen. Für die Durchführung des erfindungsgemäßen Verfahrens ist die Anwesenheit einer Schutzgruppe nicht kritisch, aber auch nicht störend. Die Einführung der Schutzgruppe kann als zusätzlicher Verfahrensschritt dem erfindungsgemäßen Verfahren vorangestellt werden. Für den Fall R⁵ = Wasserstoff, kann die Einführung der Schutzgruppe gleichzeitig oder als zusätzlicher Schritt im Anschluß an das erfindungsgemäße Verfahren durchgeführt werden. Prinzipiell kann jede Schutzgruppe verwendet werden. Bevorzugte Schutzgruppen sind die in der Literatur bekannten Schutzgruppen für Hydroxygruppen (T.W. Greene, Protective Groups in Organic Synthesis, John Wiley & Sons New York, 1981,Seite 14-71; P.J. Kocienski, Protecting Groups, Georg Thieme Verlag Stuttgart, 1994, Seite 21-94). Beispielhaft seien folgende Schutzgruppen erwähnt:
Ester, wie
   Acetat (Ac), Monochlor- bis Trichloracetate, Trifluoracetat, Phenylacetate, Triphenylmethoxyacetat, Phenoxyacetate, Halogenphenoxyacetate, Halogenalkylphenoxyacetate, Formiat, Benzoylformiat, 3-Phenylpropionat, Isobutyrat, Pivaloat (Pv), Adamantoat, Croconate, Benzoate.
Silylether, wie
   Trimethylsilyl (TMS), Triethylsilyl (TES), Methyldi-t-butylsilyl, tert-Butyldimethylsilyl (TBS oder TBDMS), tert-Butyldiphenylsilyl (TBDPS), Triphenylsilyl, Triisopropylsilyle (TIPS), Diethylisopropylsilyl (DEIPS), Isopropyldimethylsilyl (IPDMS), Thexyldimethylsilyl (TDS).
Aliphatische und aromatische Ether, wie
   Methyl (Me), Benzyl (Bn), o-Nitrobenzyl, p-Methoxybenzyle, 3,4-Dimethoxybenzylether, Trityle (Trt oder Tr), p-Methoxyphenyldiphenylmethyl (MMTr), 4, 4', 4''-tris(benzoyloxy)trityl (TBTr), di(p-Methoxyphenyl)phenylmethyl (DMTr), tert-Butyle, 9-Phenyl-9-xanthenyl (pixyl), Allyle, 2-(Trimethylsilyl)ethyl (TMSE).
Acetale (Alkoxyalkylether, Aryloxyalkylether oder Alkykloxyarylether), wie
   Methoxymethyl (MOM), Methylthiomethyl (MTM), 2-Methoxyethoxymethyl (MEM), 1-Methoxy-1-methylethyl, 2-Ethoxyethyl (EE), 4-Methoxytetrahydropyran-4-yl (MTHP), Tetrahydropyran-2-yl, 2-(Trimethylsilyl)ethoxymethyl (SEM), 3,4-dimethoxybenzyl (DMB), Benzyloxymethyl, p-Methoxybenzyl (PMB), p-Methoxybenzyloxymethyl (PMBM) und
   Methoxycarbonyle und
   Allyloxycarbonyle (Alloc), sowie davon abgeleitete, gegebenenfalls alkylierte oder halogenierte Schutzgruppen.

Besonders bevorzugte Edukte sind die 4-Oxo-Tocotrienole (R⁵ = Wasserstoff, n = 3) wie
4-Oxo-α-Tocotrienol (R¹ = R² = R³ = R⁴ = Methyl),
4-Oxo-β-Tocotrienol (R¹ = R³ = R⁴ = Methyl, R² = Wasserstoff)
4-Oxo-γ-Tocotrienol (R¹ = Wasserstoff, R² = R³ = R⁴ = Methyl) oder
4-Oxo-δ-Tocotrienol (R¹ = R² = Wasserstoff, R³ = R⁴ = Methyl)
4-Oxo-Tocotrienol (R¹ = R² = R³ = Wasserstoff, R⁴ = Methyl)
4-Oxo-2-desmethyl-Tocotrienol (R¹ = R² = R³ = R⁴ = Wasserstoff)
und die entsprechenden, am Sauerstoff geschützten (R⁵ = vorstehend erwähnten Schutzgruppen) oder derivatisierten (R⁵ = C₁-C₄-Alkyl, C₆-C₁₀-Aryl, C₇-C₁₈-Aralkyl, C₇-C₁₈-Alkylaryl oder C₁-C₂₂-Acyl) Verbindungen.

Die Reduktion der 4-Oxo-Chromanolderivate erfolgt erfindungsgemäß mit metallischem Zink in Gegenwart einer Säure oder einer Säuremischung zu den entsprechenden Chromanolderivaten der allgemeinen Formel I.
Unter Säuren werden Brönsted-Säuren sowie deren wäßrige Lösungen verstanden. Bevorzugte Brönsted-Säuren sind Mineralsäuren, wie z.B. Halogenwasserstoffsäuren, Schwefelsäuren, Salpetersäure, Phosphorsäuren, Borsäure oder Halogenoxidsäuren, insbesondere HCl, HBr, HI, HF, H₂SO₄, Methansulfonsäure, KHSO_{4,} HNO₃, HClO₄, H₃PO₄ und H₃BO₃ oder gegebenenfalls halogenierte, d.h. gegebenenfalls unabhängig voneinander mit bis zu 5 Halogenatomen wie F, Cl oder Br substituierte C₁-C₂₂-Alkancarbonsäuren, wie Ameisensäure, Essigsäure, Trifluoressigsäure, Propionsäure, Buttersäure, Citronensäure, Oxalsäure, Hexansäure, Octansäure, Decansäure (Caprinsäure), Dodecansäure (Laurinsäure), Hexadekansäure (Palmitinsäure) oder Octadecansäure (Stearinsäure).
Im Falle der wäßrigen Lösungen beträgt die Menge der Brönsted-Säure in der wäßrigen Lösung bei der Verwendung von Mineralsäuren vorzugsweise 5 bis 80 Gew.-%, besonders bevorzugt 15 bis 50 Gew.-%, bei der Verwendung der aliphatischen oder aromatischen C₁-C₂₂-Carbonsäuren vorzugsweise 10 bis 80 Gew.-%, besonders bevorzugt 35 bis 50 Gew.-%. Es können auch Mischungen der obengenannten Säuren verwendet werden.
Die Menge des zugegebenen metallischen Zn-Pulvers beträgt typischerweise 1 bis 25 Mol-äq., insbesondere 1 bis 10 Mol-äq. bezogen auf die Menge des 4-Oxo-Chromanol-Derivats.
Die Reaktionstemperatur liegt vorzugsweise bei 0 bis 160°C, insbesondere bei 20 bis 100°C. Die Dauer der Reaktion beträgt typischerweise 10 min bis 24 h.
In einer bevorzugten Ausführungsform setzt man dem Reaktionsgemisch aus Edukt (4-Oxo-Chromanolderivat), Zink und Säure ein organisches Lösungsmittel zu. Dabei kann ein mehrphasiges Gemisch entstehen. Unter organischen Lösungsmitteln werden aliphatische und aromatische Kohlenwasserstoffe, wie beispielsweise Cyclohexan, Hexan, Heptan, Oktan, Nonan, Benzol, Toluol, Xylol oder Ethylbenzol, Alkohole, wie beispielsweise Octanol, 2-Ethylhexanol oder Nonylphenol, Ether, wie beispielsweise Dibutylether oder *tert*-Butylmethylether oder C₁-C₂₂-Carbonsäuren, wie beispielsweise Hexansäure, Octansäure oder Decansäure verstanden. Auch eine Mischung daraus kann als organisches Lösungsmittel eingesetzt werden.
Bei Zusatz eines organischen Lösungsmittels ist die Verwendung einer wäßrigen Halogenwasserstoffsäure-Lösung als Säure bevorzugt. Besonders bevorzugt ist die Verwendung einer wäßrigen HCl-Lösung (Chlorwasserstoffsäurelösung).
Zusätzlich kann in einer weiteren Ausführungsform dem Reaktionsgemisch eine grenzflächen- oder phasentransferaktive Substanz, wie beispielsweise Dodecylsulfat, Dodecylamin bzw. Dodecylammoniumchlorid, Tridecylamin bzw. Tridecylammoniumchlorid, Methyltrioctylammoniumchlorid, Benzyltrimethylammoniumbromid oder Tetraphenylphosphoniumbromid zugemischt werden. Als weitere Additive können insbesondere Halogenid-Salze wie beispielsweise Lithiumchlorid, Natriumchlorid, Magnesiumchlorid, Kaliumbromid, Cäsiumfluorid oder Tetramethylammoniumchlorid vorteilhaft zugemischt werden. Vorteilhafterweise kann das Verfahren unter Ultraschall durchgeführt werden.

In einer weiteren bevorzugten Ausführungsform können die C₁-C₂₂-Alkancarbonsäuren, die als Brönsted-Säuren verwendet werden, selbst als Lösungsmittel dienen. Dabei setzt man die 4-Oxo-Chromanolderivate der allgemeinen Formel II in einer C₁-C₂₂-Alkancarbonsäure, wie vorstehend erwähnt, ohne zusätzliche Zugabe eines organischen Lösungsmittels mit Zink um. Bevorzugte Säuren dieser Ausführungsform sind Ameisensäure, Essigsäure, Propionsäure, Buttersäure, Pentansäure, Hexansäure, Heptansäure, Octansäure, Decansäure, Dodecansäure, Palmitinsäure oder Stearinsäure. Besonders bevorzugt ist die Verwendung von Essigsäure.

Wie vorstehend beschrieben, kann der Schritt der Einführung einer Schutzgruppe, bzw. die Derivatisierung der Hydroxygruppe (R⁵) auch gleichzeitig oder im Anschluß an das erfindungsgemäßen Verfahren durchgeführt werden. Bevorzugt ist dabei aufgrund der pharmakologischen Relevanz die Einführung einer Estergruppe.

Zur Herstellung von Chromanol-Estern der allgemeinen Formel Ia 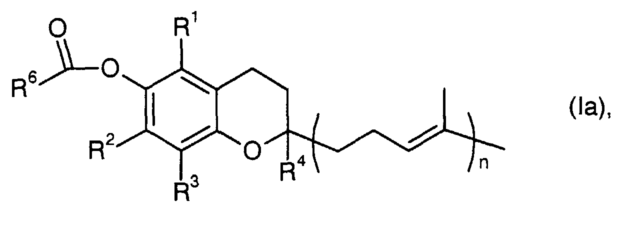 wobei R¹, R² R³, R⁴ und n die vorstehend beschriebene Bedeutung haben und
- R⁶: Wasserstoff,
einen C₁-C₂₁-Alkylrest, wie Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl oder Heneicosyl oder
einen C₂-C₂₁-Alkenylrest, wie Ethenyl , 1-Propenyl, 2-Propenyl, 8-Heptadecenyl, 8,11-Heptadecadienyl oder 8,11,14-Heptadecatrienyl
bedeutet, kann daher auch ein Gesamtverfahren durchgeführt werden, das dadurch gekennzeichnet ist, daß man die Chromanol-Derivate der Formel I mit R⁵ = Wasserstoff (Ib) 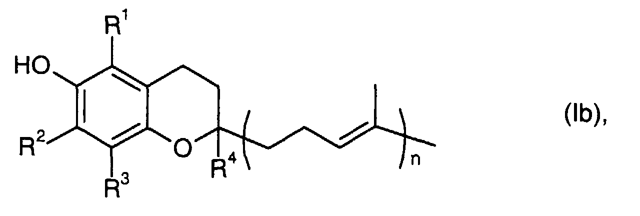 nach dem erfindungsmäßigen Verfahren durch Reduktion des entsprechenden 4-Oxo-Chromanol-Derivates der allgemeinen Formel II (R⁵ = Wasserstoff) herstellt und diese in üblicher Weise verestert, in dem man mit einem aliphatischen C₁-C₂₂-Carbonsäurederivat der allgemeinen Formel III

R⁶―COX (III)

umsetzt, wobei
- X: eine durch die Hydroxygruppe des Chromanol-Derivats der Formel Ib verdrängbare Abgangsgruppe darstellt.

Die Veresterung erfolgt demnach in an sich bekannter Weise durch Umsetzung des Chromanolderivats der Formel Ib mit C₁-C₂₂-Alkancarbonsäuren (R⁶ = C₁-C₂₁-Alkylrest, X = OH) wie vorstehend als Brönsted-Säuren beschrieben, C₂-C₂₂-Alkencarbonsäuren (R⁶ = C₂-C₂₁-Alkenylrest, X = OH), wie Acrylsäure, Methacrylsäure, Crotonsäure, Sorbinsäure, Ölsäre, Linolsäure, Linolensäure, Eicosapentaensäure oder Docosahexaensäure oder mit den entsprechenden aliphatischen C₁-C₂₂-Carbonsäurederivaten (Veresterungsmittel), d.h. den entsprechenden C₁-C₂₂-Alkancarbonsäurederivaten oder C₂-C₂₂-Alkencarbonsäurederivaten. Unter den aliphatischen C₁-C₂₂-Carbonsäurederivaten der Formel III werden Carbonsäureanhydride, Carbonsäurehalogenide, wie Carbonsäurechloride, -fluoride oder -bromide oder Carbonsäureaktivester der C₁-C₂₂-Alkancarbonsäuren bzw. C₂-C₂₂-Alkencarbonsäuren verstanden.

Für Carbonsäureaktivester seien beispielhaft Carbonsäureester, wie Carbonsäure-N-Hydroxysuccinimide, Phenolester und Halophenolester wie Pentafluorphenolester und Trichlorphenolester genannt.

Als Abgangsgruppe X seien beispielhaft dementsprechend Halogenide wie F⁻, Cl⁻, Br⁻, Carboxylate wie Acetat oder Propiat oder Alkoholate wie Succinimid-N-Hydroxylat, Phenolat, Halophenolate wie Pentafluorphenolat, Trichlorphenolate oder Benzotriazol-1-Hydroxylat genannt.

Bevorzugte Veresterungsmittel sind Acetylierungsmittel, wie beispielsweise Essigsäureanhydrid oder Essigsäurechlorid.

In einer bevorzugten Ausführungsform werden zur Herstellung von Chromanol-C₁-C₂₂-Alkancarbonsäureester die Chromanol-Derivate der Formel I mit R⁵ = Wasserstoff (Ib) nach dem erfindungsmäßigen Verfahren unter Verwendung von C₁-C₂₂-Alkancarbonsäuren als Brönstedsäuren oder organisches Lösungsmittel hergestellt, wobei diese C₁-C₂₂-Alkancarbonsäuren gleichzeitig, vorteilhaft in situ, als Veresterungsmittel verwendet werden.

Das erfindungsgemäße Verfahren dient zur vereinfachten Darstellung von Chromanolderivaten durch Reduktion der entsprechenden 4-Oxo-Chromanolderivate. Gegenüber dem Stand zeichnet es sich durch folgende Vorteile aus:
- Das Verfahren läßt sich einfach in einem Verfahrensschritt durchführen.
- Das Verfahren erfordert auch im technischen Maßstab keine hohen Sicherheitsvorkehrungen.
- Das Verfahren erfordert technisch gut verfügbare und wohlfeile Einsatzstoffe.

Die nachstehenden Beispiele erläutern die Erfindung

### Beispiele 1 bis 6

### Herstellung von γ-Tocotrienol durch Reduktion von 4-Oxo-γ-Tocotrienol in n-Heptan mit Zn und einer wäßrigen HCl-Lösung

Entsprechend den nachstehenden Versuchsbedingungen wurden die in Tabelle 1 und 2 zusammengestellten Versuchsergebnisse erzielt.

4-Oxo-γ-Tocotrienol (1 g bis 10.3 g, 2,36 mmol bis 24 mmol) wurde durch Erwärmen in n-Heptan (Beispiel 1-4), Toluol (Beispiel 5) oder Octansäure (Beispiel 6) gelöst. Nach Abkühlen auf Raumtemperatur wurde unter Rühren eine wäßrige HCl-Lösung (20 bis 24 Gew.-%, bezogen auf die wäßrige Lösung) dazugegeben. Anschließend wurde innerhalb von 10 Minuten Zinkpulver (5 bzw. 10 Moläquivalente (Mol-äq.) bezogen auf 4-Oxo-γ-Tocotrienol) eingetragen und das Reaktionsgemisch die in den Tabellen 1 und 2 angegebene Zeit weitergerührt. Das Zink wurde mit Celite® (Warenzeichen der Manville Corp., USA, für Filterhilfsmittel zur Trennung u. Reinigung von Flüssigkeiten. Celite® bestehen aus Kieselgur verschiedener Korngrößen) über eine Nutsche abfiltriert und mit n-Heptan (100 ml) nachgewaschen. Die organische Phase wurde gewaschen, über Na₂SO₄ getrocknet, eingeengt und die Reinheit per GC durch Flächenintegration bestimmt.
Das Rohprodukt wurde in Beispiel 1 und 2 durch Flash-Chromatographie mit Methyl-t-butylether/Heptan als Eluent über Kieselgel bzw. durch Kugelrohrdestillation gereinigt.

**Tabelle 1**

| | Beispiel 1 | Beispiel 2 | Beispiel 3 | Beispiel 4 |
|---|---|---|---|---|
| 4-Oxo-γ-T. [g] | 1,0 | 5,0 | 10,3 | 10,0 |
| 4-Oxo-γ-T. [mmol] | 2,3 | 11,8 | 24,0 | 22,7 |
| Zn [Mol-äq.] | 10 | 5 | 5 | 5 |
| Heptan [ml] | 10 | 80 | 100 | 100 |
| HCl (aqu.) [ml] | 20 | 50 | 183 | 200 |
| HCl in wäßriger Lösung [Gew.-%] | 20 | 20 | 22 | 24 |
| Temperatur [°C] | RT | 60 | 40 | 40 |
| Dauer [h] | 1,5 | 2 | 1 | 1 |
| Rohausbeute [g] | 0,96 | 4,64 | 9,88 | 9,27 |
| Reinheit [%] | 77 | - | 82 | 81 |
| Ausbeute vor Reinigung [%] | 76 | - | 82 | 79 |
| Reinigung durch | Flash-Chromatographie | Kugelrohrdestillation | - (*) | - |
| Ausbeute nach Reinigung [%] | 63 | 52 | - | - |
| Verwendete Abkürzungen: T. = Tocotrienol aqu. = wäßrige Lösung | | | | |

| | | | | |
|---|---|---|---|---|
| (*) Weitere Umsetzung ohne Reinigung in Beispiel 12 | | | | |

**Tabelle 2**

| | Beispiel 5 | Beispiel 6 |
|---|---|---|
| 4-Oxo-γ-T. [g] | 2,07 | 2,02 |
| 4-Oxo-γ-T. [mmol] | 4,83 | 4,71 |
| Zn [Mol-äq.] | 5 | 5 |
| Lösungsmittel | Toluol | Octansäure |
| Menge an Lösungsmittel [ml] | 20 | 20 |
| HCl(aqu.) [ml] | 40 | 40 |
| HCl in wäßriger Lösung [Gew.-%] | 22 | 22 |
| Temperatur [°C] | 40 | 40 |
| Dauer [h] | 1,0 | 3,0 |
| Rohausbeute [g] | 1,83 | 1,1 |
| Reinheit [%] | 82 | 62 |
| Ausbeute vor Reinigung [%] | 76 | 38 |
| Verwendete Abkürzungen: T. = Tocotrienol aqu. = wäßrige Lösung | | |

### Beispiel 7

### Herstellung von γ-Tocotrienol durch Reduktion von 4-Oxo-γ-Tocotrienol in Oktan mit Zn und einer wäßrigen HCl-Lösung unter Zusatz von Dodecylamin als grenzflächenaktiver Substanz

Die Versuchsdurchführung erfolgte analog zu den Beispielen 1 bis 6 unter Zusatz von Dodecylamin als grenzflächenaktiver Substanz und Verwendung von Octan als Lösungsmittel. Unter den Reaktionsbedingungen liegt das zugegebene Dodecylamin als Dodecylammoniumchlorid vor. Im Unterschied zu den Beispielen 1 bis 4 erfolgte die Reaktionsdurchführung unter Ultraschall und nicht unter Rühren. Tabelle 3 faßt die Reaktionsbedingungen und Ergebnisse zusammen.

**Tabelle 3**

| | |
|---|---|
| 4-Oxo-γ-T. [g] | 0,83 |
| 4-Oxo-γ-T. [mol] | 1,9 |
| Zn [Mol-äq.] | 10 |
| Oktan [ml] | 10 |
| HCl(aqu.) [ml] | 30 |
| HCl in wäßriger Lösung [Gew.-%] | 20 |
| grenzflächenaktive Substanz | Dodecylamin |
| Menge der grenzflächenaktiven Substanz [g] | 0,05 |
| Temperatur [°C] | 45 |
| Dauer [min] | 20 |
| Rohausbeute [g] | 0, 77 |
| Reinheit [%] | 53 |
| Ausbeute vor Reinigung in [%] | 52 |
| Verwendete Abkürzungen: T. = Tocotrienol aqu. = wäßrige Lösung | |

### Beispiel 8

### Herstellung von γ-Tocotrienol durch Reduktion von 4-Oxo-γ-Tocotrienol in Heptan mit Zn und einer wäßrigen H₂SO₄-Lösung unter Zusatz einer grenzflächenaktiven Verbindung

Die Versuchsdurchführung erfolgte analog zu den Beispielen 1 bis 6 unter Verwendung einer wäßrigen H₂SO₄-Lösung (50 Gew.-%, bezogen auf die wäßrige Lösung) und unter Zusatz einer grenzflächenaktiven Verbindung. In Tabelle 4 sind die Reaktionsbedingungen und die Ergebnisse zusammengestellt.

**Tabelle 4**

| | |
|---|---|
| 4-Oxo-γ-T. [g] | 2,1 |
| 4-Oxo-γ-T. [mmol] | 4,7 |
| Zn [Mol-äq.] | 8,5 |
| Heptan [ml] | 20 |
| H₂SO₄(aqu.) [ml] | 60 |
| H₂SO₄ in wäßriger Lösung [Gew.-%] | 50 |
| Grenzflächenaktive Verbindung | Dodecylamin |
| Menge der grenzflächenaktiven Verbindung [g] | 0,08 |
| Temperatur [°C] | 55 |
| Dauer [h] | 12 |
| Rohausbeute [g] | 1,5 |
| Umsatz [%] | 98 |
| Selektivität [%] | 30 |
| Verwendete Abkürzungen: T. = Tocotrienol aqu. = wäßrige Lösung | |

### Beispiel 9

### Herstellung von γ-Tocotrienol durch Reduktion von 4-Oxo-γ-Tocotrienol in Heptan mit Zn und einer wäßrigen Citronensäure-Lösung

4-Oxo-γ-Tocotrienol (0,81 g, 1,9 mmol) wurde bei 60°C in n-Heptan gelöst und unter Rühren eine wäßrige Citronensäurelösung (20 ml, 40 Gew.-%, bezogen auf die wäßrige Lösung) dazugegeben. Anschließend wurde Zinkpulver (1,3 g, 10 Mol-äq. bezogen auf 4-Oxo-γ-Tocotrienol) eingetragen und das Reaktionsgemisch 6 h bei 60°C und anschließend 2 h bei 75°C gerührt. Das Zink wurde mit Celite über eine Nutsche abfiltriert, die organische Phase abgetrennt, mit Wasser gewaschen und über Na₂SO₄ getrocknet. Gaschromatographisch wurde der Umsatz auf 24 % bestimmt (76 % 4-Oxo-γ-Tocotrienol wurden zurückgewonnen). Die Selektivität der Reaktion betrug 86 %.

### Beispiel 10

### Herstellung von γ-Tocotrienol durch Reduktion von 4-Oxo-γ-Tocotrienol in n-Nonan mit Zn und einer Mischung aus einer wäßrigen Citronensäure- und HCl-Lösung

4-Oxo-γ-Tocotrienol (2,05 g, 4,78 mmol) wurde in warmen (50°C) Nonan (35 ml) gelöst und mit einer wäßrigen Citronensäurelösung (300 ml, 40 Gew.-%, bezogen auf die wäßrige Lösung) unter Rühren auf 100°C erwärmt. Im Laufe von 5 h wurde portionsweise Zinkpulver (3,1 g, 10 Mol-äq. bezogen auf 4-Oxo-γ-Tocotrienol) unter Rühren zugesetzt. Anschließend wurde eine wäßrige Salzsäurelösung (Chlorwasserstoffsäurelösung) (30 ml, 24 Gew.-%, bezogen auf die wäßrige Lösung) zugegeben und im Laufe von 4 h weiteres Zinkpulver (3,1 g, 10 Mol-äq.) portionsweise zugegeben. Nach Abkühlen auf Raumtemperatur wurde die organische Phase abgetrennt, mit Wasser gewaschen, über Na₂SO₄ getrocknet und eingeengt. Bei vollständigem Umsatz wurde gaschromatographisch eine Ausbeute von 62 % γ-Tocotrienol bestimmt.

### Beispiel 11

### Herstellung von γ-Tocotrienol durch Reduktion von 4-Oxo-γ-Tocotrienol mit Zn und Eisessig

4-Oxo-γ-Tocotrienol (2,07 g, 4,83 mmol) wurden in 50 ml Eisessig gelöst und auf Rückfluß( ca. 80°C) erwärmt. Es wurden portionsweise 14,2 Mol-äq. Zinkpulver eingetragen und 14 h am Rückfluß gerührt. Der Fortgang der Reaktion wurde dünnschicht- und gaschromatographisch verfolgt. Bei einem Umsatz von 62 % wurde abgebrochen, restliches Zinkpulver abfiltriert und das Filtrat zwischen Heptan (100 ml) und Wasser (100 ml) verteilt. Die heptanische Phase wurde mit Wasser gewaschen, über Na₂SO₄ getrocknet und eingeengt. Chromatographie über Kieselgel mit Methyl-t-Butyl-ether/Heptan als Eluent ergab 0,51 g (1,24 mmol) γ-Tocotrienol und 0,06 g Tocotrienolacetat. Nicht umgesetztes 4-Oxo-γ-Tocotrienol (0,61 g) wurden zurückgewonnen.

### Beispiel 12

### Herstellung von γ-Tocotrienolacetat durch Acetylierung von γ-Tocotrienol mit Essigsäureanhydrid

γ-Tocotrienol (Rohprodukt, hergestellt nach Beispiel 3, 4,8 g) wurde mit 10 ml Essigsäureanhydrid 3,5 h am Rückfluß erhitzt und anschließend im Vakuum von Essigsäure und Essigsäureanhydrid befreit. Flash-Chromatographie des Rohprodukts mit Ethylacetat/Heptan als Eluent über Kieselgel ergab 3,7 g γ-Tocotrienolacetat.

### Beispiele 13 und 14

### Herstellung von α-Tocotrienol durch Reduktion von 4-oxo-α-Tocotrienol in Tolual mit Zink und einer wäßrigen Salzsäurelösung bzw. in Cyclohexan mit Zink und einer wäßrigen Ameisensäurelösung.

4-Oxo-α-Tocotrienol (0,26 g, 0,6 mmol) wurde bei 40°C in Toluol (Bsp. 13) oder Cyclohexan (Bsp. 14) gelöst. Unter Rühren wurde die wäßrige HCl-Lösung (22%ig) (Bsp. 13) oder die wäßrige Ameisensäurelösung (80%ig) (Bsp. 14) dazugegeben. Anschließend wurde innerhalb 8¹/₂ Stunden Zinkpulver (13 bzw. 15 Mol-äq, bezogen auf 4-Oxo-α-Tocotrienol) eingetragen und das Reaktionsgemisch 5 Stunden bei 40°C und dann 7 Stunden bei 65°C gerührt. Der Reaktionsfortgang wird durch Probennahme und Dünnschichtchromatographie verfolgt. Unumgesetztes Zink wurde mit Celite über eine Nutsche abfiltriert und mit n-Heptan nachgewaschen. Die organische Phase wurde mit Wasser gewaschen, über Na₂SO₄ getrocknet und eingeengt. Tabelle 5 faßt die Reaktionsbedingungen und Ergebnisse zusammen.

**Tabelle 5**

| | Beispiel 13 | Beispiel 14 |
|---|---|---|
| 4-Oxo-α-T. [g] | 0,26 | 0,26 |
| 4-Oxo-α-T. [mmol] | 0,60 | 0,60 |
| Zink [Mol-äq.] | 13 | 15 |
| Lösungsmittel | Toluol | Cyclohexan |
| Menge an Lösungsmittel [ml] | 3 | 3 |
| Säure (aqu.) | Salzsäure | Ameisensäure |
| Säuremenge [ml] | 5 | 5 |
| Konz. der Säure in wäßriger Lösung [Gew.-%] | 22 % HCl | 80 % HCOOH |
| Temperatur [°C] | 40 u. 65 | 40 u. 65 |
| Dauer (Std.) | 12 | 12 |
| Rohausbeute [g] | 0,10 | 0,10 |
| Umsatz | 91 % | 88 % |
| Selektivität | 93 % | 93 % |

### Beispiele 15 und 16

### Herstellung von δ-Tocotrienol durch Reduktion von 4-Oxo-δ-Tocotrienol in Toluol mit Zink und einer wäßrigen Salzsäurelösung bzw. in Cyclohexan mit Zink und einer wäßrigen Ameisensäurelösung

4-Oxo-δ-Tocotrienol (0,20 g, 0,5 mmol) wurde durch Erwärmen in Toluol (Bsp. 15) oder Cyclohexan (Bsp. 16) gelöst. Unter Rühren wurde die wäßrige HCl-Lösung (22%ig) (Bsp. 15) oder die wäßrige Ameisensäurelösung (80%ig) (Bsp. 16) dazugegeben. Anschließend wurde innerhalb 0,5 Std. (Bsp. 15) bzw. 7 Std. (Bsp. 16) Zinkpulver (6 bzw. 10 Mol-äq., bezogen auf 4-Oxo-δ-Tocotrienol) eingetragen und das Reaktionsgemisch 2 Std. (Bsp. 15) bzw. 9 Std. (Bsp. 16) bei 60°C gerührt. Das Zink wurde mit Celite über eine Nutsche abfiltriert und mit n-Heptan nachgewaschen. Die organische Phase wurde mit Wasser gewaschen, über Na₂SO₄ getrocknet und eingeengt. In Tabelle 6 sind die Reaktionsbedingungen und die Ergebnisse zusammengestellt.

**Tabelle 6**

| | Beispiel 15 | Beispiel 16 |
|---|---|---|
| 4-Oxo-δ-T. [g] | 0,20 | 0,20 |
| 4-Oxo-δ-T. [mmol] | 0,50 | 0,50 |
| Zink [Mol-äq.] | 6 | 10 |
| Lösungsmittel | Toluol | Cyclohexan |
| Menge an Lösungsmittel [ml] | 3 | 3 |
| Säure (aqu.) | Salzsäure | Ameisensäure |
| Säuremenge (aqu.) [ml] | 5 | 5 |
| Konz. der Säure in wäßriger Lösung [Gew. -%] | 22 % HCl | 80 % HCOOH |
| Temperatur [°C] | 60 | 60 |
| Dauer (Std.) | 2 | 9 |
| Rohausbeute [g] | 0,16 | 0,11 |
| Umsatz | 100 % | 99,5 % |
| Selektivität | 93 % | 56 % |

### Beispiel 17

### Herstellung von γ-Tocotrienol durch Reduktion von 4-Oxo-γ-Tocotrienol in Cyclohexan mit Zink und einer wäßrigen Ameisensäurelösung.

4-Oxo-γ-Tocotrienol (100,3 g, 0,24 mol) wurde bei 65°C in 500 ml Cyclohexan und 1 Liter Ameisensäure (aqu., 80%ig) gelöst. Unter Rühren wurde in 9¹/₂ Stunden Zinkpulver (160 g, 2,45 mol) eingetragen und das Reaktionsgemisch bei 65°C 12 Stunden gerührt. Nach 4 Stunden Rührzeit wurde nochmals 1 Liter Ameisensäure (aqu., 80%ig) zugegeben. Die organische Phase wurde zweimal mit Wasser und dann zweimal mit einem Methanol-/Wasser-Gemisch (1:1) gewaschen und eingeengt. Ausbeute: 96,1 g rohes γ-Tocotrienol. 91 g des Rohproduktes wurden bei 200°C/<0,01 mbar einer Molekulardestillation unterworfen und ergaben 86 g γ-Tocotrienol als gelbliches Öl.

## Patentansprüche

1. Verfahren zur Herstellung von Chromanol-Derivaten der allgemeinen Formel I 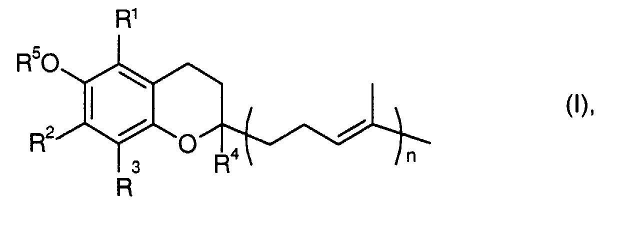 wobei
n 1 bis 10,
R^{1,} R^{2,} R^{3,} R⁴ unabhängig voneinander Wasserstoff oder einen C₁-C₄-Alkylrest und
R⁵ Wasserstoff, einen C₁-C₄-Alkyl-, C₆-C₁₀-Aryl-, C₇-C₁₈-Aralkyl-, C₇-C₁₈-Alkylaryl-, C₁-C₂₂-Acylrest oder eine Schutzgruppe der Hydroxygruppe
bedeuten, durch Reduktion der 4-Oxo-Chromanol-Derivate der allgemeinen Formel II 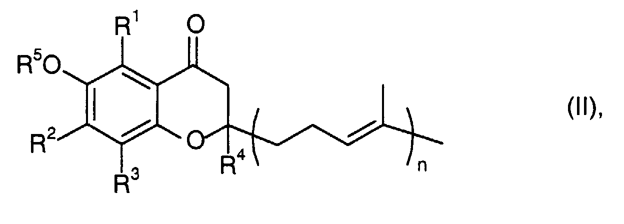 dadurch gekennzeichnet, daß man die 4-Oxo-Chromanol-Derivate der Formel II mit metallischem Zink in Gegenwart einer Säure oder einer Säuremischung umsetzt.

2. Verfahren nach Anspruch 1, dadurch gekennzeichnet, daß man dem Reaktionsgemisch ein organisches Lösungsmittel zusetzt.

3. Verfahren nach Anspruch 1 oder 2, dadurch gekennzeichnet, daß man als Säure eine Halogenwasserstoffsäure oder deren wäßrige Lösung verwendet.

4. Verfahren nach Anspruch 1 oder 2, dadurch gekennzeichnet, daß man als Säure Chlorwasserstoffsäure oder deren wäßrige Lösung verwendet.

5. Verfahren nach Anspruch 1, dadurch gekennzeichnet, daß man als Säure eine C₁-C₂₂-Alkancarbonsäure verwendet und diese auch als organisches Lösungsmittel einsetzt.

6. Verfahren nach Anspruch 5, dadurch gekennzeichnet, daß man als C₁-C₂₂-Alkancarbonsäure Essigsäure verwendet.

7. Verfahren zur Herstellung von Chromanol-Estern der allgemeinen Formel Ia 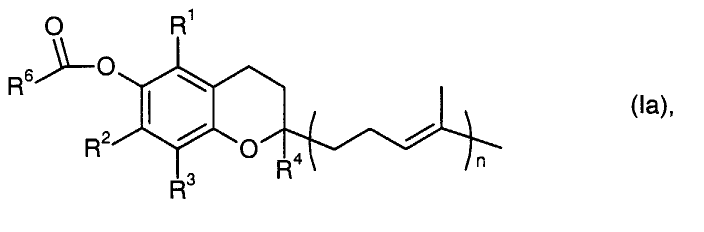 wobei
n 1 bis 10,
R^{1,} R^{2,} R^{3,} R⁴ unabhängig voneinander Wasserstoff oder einen C₁-C₄-Alkylrest und
R⁶ Wasserstoff, einen C₁-C₂₁-Alkyl- oder einen C₂-C₂₁-Alkenylrest
bedeuten, dadurch gekennzeichnet, daß man die Chromanol-Derivate der Formel I mit R⁵ = Wasserstoff (Ib) 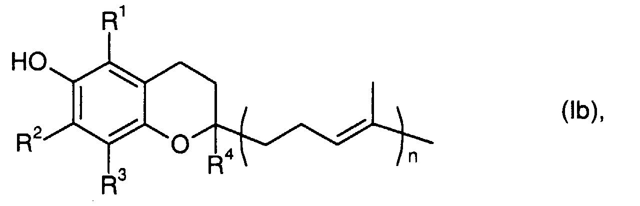 gemäß einem Verfahren der Ansprüche 1 bis 6 herstellt und in üblicher Weise verestert, indem man mit einem aliphatischen C₁-C₂₂-Carbonsäurederivat der allgemeinen Formel III
R⁶―COX (III)
umsetzt, wobei
X eine durch die Hydroxygruppe des Chromanol-Derivats der Formel Ib verdrängbare Abgangsgruppe darstellt.

8. Verfahren nach Anspruch 7, dadurch gekennzeichnet, daß man die Chromanol-Derivate der Formel Ib gemäß Anspruch 5 herstellt und mit der C₁-C₂₂-Alkancarbonsäure, die im Reaktionsgemisch des Reduktionsschrittes vorliegt, umsetzt.
